# EUROPEAN PATENT SPECIFICATION

(11) **EP 2 934 331 B1**
(45) Date of publication and mention of the grant of the patent: **06.03.2019**
(21) Application number: 13864632.8
(22) Date of filing: 19.12.2013
(51) Int. Cl.: A61B 8/12

(54) **INTRALUMINAL IMAGING DEVICE**
INTRALUMINALE BILDGEBUNGSVORRICHTUNG
DISPOSITIF INTRALUMINAL D'IMAGÉRIE

(30) Priority: 20.12.2012 US 201261739835 P
(43) Date of publication of application: 28.10.2015
(73) Proprietor: Stigall, Jeremy, San Diego, CA 92130 (US); Minas, Maritess, San Diego, CA 92130 (US)
(72) Inventor: Stigall, Jeremy, San Diego, CA 92130 (US); Minas, Maritess, San Diego, CA 92130 (US)
(74) Representative: de Haan, Poul Erik
(86) International application number: PCT/US2013/076587
(87) International publication number: WO 2014/100422

(56) References cited:
- WO-A1-91/01156
- US-A- 5 387 193
- US-A- 5 514 128
- US-A- 6 078 831
- US-A1- 2003 093 059
- US-A1- 2005 215 942
- US-B2- 7 294 124
- US-B2- 7 294 124

## Description

### FIELD OF THE INVENTION

The invention relates to elongate intraluminal devices such as catheters. The invention provides enhanced designs that improve control and deliverability, e.g., to tortuous vasculature.

### BACKGROUND

It is estimated that nearly one million endovascular procedures are performed each year in the United States. See Satiani et al., "Predicted Shortage of Vascular Surgeons in the United States," J. Vascular Surgery, vol. 50 (2009) p. 946-952. Endovascular procedures typically begin by placing an introducer into an access artery (e.g., brachial, femoral, radial) through which a medical device can be passed, providing access to vasculature and organs throughout the body. Because the devices enter the body through a small hole and then travel a distance to the treatment site, the devices are typically quite long (100 cm or greater) and very slender (5 mm or less).

A common complaint regarding endovascular devices is that the distal end of the device (i.e., where treatment is administered) does not follow manipulations at the proximal end (i.e., outside of the patient). For example, when the device is pushed further into the introducer, the distal end (observed with angioscopy) does not move the corresponding distance ("push"). Other times, when the proximal end is torqued, the distal end does not rotate as expected ('track"). Pushing and tracking are crucial in negotiating difficult curves or obstructions within the vasculature. When an endovascular device fails to push and track accurately, the procedure goes slower, which requires the patient to be exposed to greater amounts of anesthesia. Additionally, serious errors in pushing and tracking can require the use of additional fluoroscopy and contrast to verify that there is not an unexpected occlusion causing the error.

Intraluminal devices that have good pushing and tracking qualities may be hard to work with, however. A catheter that pushes and rotates as expected is often quite stiff, which can make it difficult to deliver through tortuous turns in the vasculature. Stiff catheters also have a greater likelihood of perforating a vessel or causing other mechanical damage. Additionally, stiff catheters can actually result in greater pushing resistance at the distal end of a guide wire because the catheter has difficulty conforming to the contours of the guide wire. Resistance at the distal end of a guide wire can make it difficult to place the distal tip of the catheter in a desired location.

US 6 078 831 discloses an imaging catheter comprising
- a proximal segment comprising a hypotube comprising a skived terminal portion, and
- a distal segment comprising an imaging element.

This imaging catheter can be considered to form the closest prior art for the invention.

Thus, there remains a need for intraluminal devices having the correct blend of stiffness and compliance.

### SUMMARY

The disclosed intravascular devices deliver the needed balance between stiffness and compliance by adding a variable stiffness element to transition an intraluminal device from a stiffer proximal to a supple distal end. As disclosed herein, a variable stiffness element, composed of multiple structures, or composed of one structure with multiple mechanical features, is located in the midsection of the catheter. The variable stiffness element provides a smooth transition from a metal hypotube at the proximal end to the flexible distal end, providing better push and tracking characteristics but reducing the likelihood of kinks. The disclosed elements are well-suited for imaging catheters which require a flexible distal end with a narrow diameter but also good pushing and tracking characteristics.

An intraluminal device of the invention includes a proximal segment, a distal segment and a midsection between the proximal and distal segments. The proximal segment will typically include a hypotube that is at least 10 cm long. In some embodiments, i.e., imaging catheters, the distal segment will include an imaging element used to acquire intraluminal images. The imaging element may produce acoustic waves for ultrasound imaging.

As discussed in detail below, variable stiffness elements include structures such as spiral cut tubes, skives, and tapered wires. The structures can be assembled into a variable stiffness element, or a single element can be fabricated having multiple features. Because the elements are hollow, they allow passage of microcables that connect imaging elements, etc., at the distal end of the device to control electronics, etc., at the proximal end of the device. The microcables can also be configured to deliver signals, torque, and/or mechanical translation to an imaging element at the distal end of the catheter. The variable stiffness elements may be constructed from metal, such as stainless steel or nitinol, or polymers.

One element that is fabricated from a single piece of material includes a hypotube having an outer diameter of less than 0.5 cm, and includes an uninterrupted cylindrical portion having a length of at least 10 cm, a tapered portion having a length of at least 2 cm, and a spiral-cut portion having a length of at least 1 cm. Another element that is fabricated from a single piece of material includes a hypotube having an outer diameter of less than 0.5 cm, and includes an uninterrupted cylindrical portion having a length of at least 10 cm, a spiral-cut portion having a length of at least 1 cm, and an elongated skive portion having a length of at least 1 cm.

Catheters including the disclosed variable stiffness constructed can be used to evaluate, modify, and treat a variety of tissues. Catheters of the invention can be prepared having a variety of functionality including, but not limited to, IVUS imaging, OCT imaging, ablation, aspiration, irrigation, energy delivery, drug delivery, therapy delivery, device delivery, tissue biopsy, and combinations thereof.

### BRIEF DESCRIPTION OF THE DRAWINGS

FIG. 1 depicts proximal, mid, and distal sections of a prior art catheter;
FIG. 2 depicts a catheter of the invention having a variable stiffness midsection;
FIG. 3 depicts a catheter having a variable stiffness midsection and a phased array imaging sensor;
FIG. 4 depicts a catheter having a variable stiffness midsection and a rotating pullback imaging sensor;
FIG. 5 shows a hypotube having a skive used to construct a variable stiffness element;
FIG. 6 shows a tapered stiffening wire used to construct a variable stiffness element;
FIG. 7A shows a side view of a variable stiffness element of the invention;
FIG. 7B shows an end view of a variable stiffness element of the invention;
FIG. 8A shows a side view of a variable stiffness element of the invention, including a microcable passing through the hypotube;
FIG. 8B shows an end view of a variable stiffness element of the invention, including a microcable passing through the hypotube;
FIG. 9 shows a phased array imaging catheter employing a variable stiffness element;
FIG. 10 shows a detailed view of the variable stiffness midsection assembly of FIG. 9;
FIG. 11 shows a spiral-cut hypotube used to construct a variable stiffness element;
FIG. 12A shows a side view of a variable stiffness element of the invention;
FIG. 12B shows an end view of a variable stiffness element of the invention;
FIG. 13A shows a side view of a variable stiffness element of the invention, including a microcable passing through the hypotube;
FIG. 13B shows an end view of a variable stiffness element of the invention, including a microcable passing through the hypotube;
FIG. 14 shows a phased array imaging catheter employing a variable stiffness element;
FIG. 15 shows a detailed view of the variable stiffness midsection assembly of FIG. 14;
FIG. 16A shows a variable stiffness element including a partially-spiral-cut hypotube with an extended skive;
FIG. 16B shows a detailed view of the partially-spiral-cut hypotube of FIG. 16A;
FIG. 16C shows a detailed view of the extended skive of FIG. 16A;
FIG. 17A shows a side view of a variable stiffness element including a partially-spiral-cut hypotube with an extended skive, including a microcable passing through the hypotube;
FIG. 17B shows an end view of a variable stiffness element including a partially-spiral-cut hypotube with an extended skive, including a microcable passing through the hypotube;
FIG. 18 shows a phased array imaging catheter employing a variable stiffness element;
FIG. 19 shows a detailed view of the variable stiffness midsection assembly of FIG. 18;
FIG. 20 shows a variable stiffness element including a tapered hypotube having a spiral-cut portion;
FIG. 21 shows a variable stiffness element including a tapered hypotube having a spiral-cut portion, including a microcable passing through the hypotube;
FIG. 22 shows a phased array imaging catheter employing a variable stiffness element;
FIG. 23 shows a detailed view of the variable stiffness midsection assembly of FIG. 23;
FIG. 24 shows a hypotube with a spiral cut used to construct a variable stiffness element;
FIG. 25 shows a tapered stiffening wire used to construct a variable stiffness element;
FIG. 26A shows a side view of a variable stiffness element of the invention;
FIG. 26B shows an end view of a variable stiffness element of the invention;
FIG. 27A shows a side view of a variable stiffness element of the invention, including a microcable passing through the hypotube;
FIG. 27B shows an end view of a variable stiffness element of the invention, including a microcable passing through the hypotube;
FIG. 28 shows a phased array imaging catheter employing a variable stiffness element;
FIG. 29 shows a detailed view of the variable stiffness midsection assembly of FIG. 28.

### DETAILED DESCRIPTION

The invention discloses structures that provide a desired combination of stiffness and flexibility in an intraluminal device. The devices generally include variable stiffness elements that exhibit a gradated relationship between load and displacement as a function of length. That is, one end of a variable stiffness element is rather stiff, and the end is rather flexible, with a smooth transition in stiffness from end to end. Using the disclosed devices, including variable stiffness elements, a surgeon will achieve better pushing and tracking while retaining the desired flexibility at the distal end of the device. Accordingly, the devices of the invention will speed intravascular procedures, resulting in less anesthesia for the patient. The devices will also reduce the rates of complication due to kinking and/or vessel perforation.

As discussed in the Background, producing the desired combination of stiffness and flexibility in an intraluminal device can be tricky. Extending the length of mechanical elements that increase stiffness and tracking, such as metal tubes and wires, make the overall device less flexible, diminishing performance at the distal (i.e., treatment) end of the device, where flexibility is at a premium. The conflict between stiffness and flexibility is compounded as device manufacturers add more functionality to intraluminal devices, e.g., catheters. For example, imaging catheters require better push performance so that the distal tip can be delivered past lesions to be imaged, while at the same time the distal end must smoothly transition through tortuous vasculature without binding the imaging elements or the associated electronics.

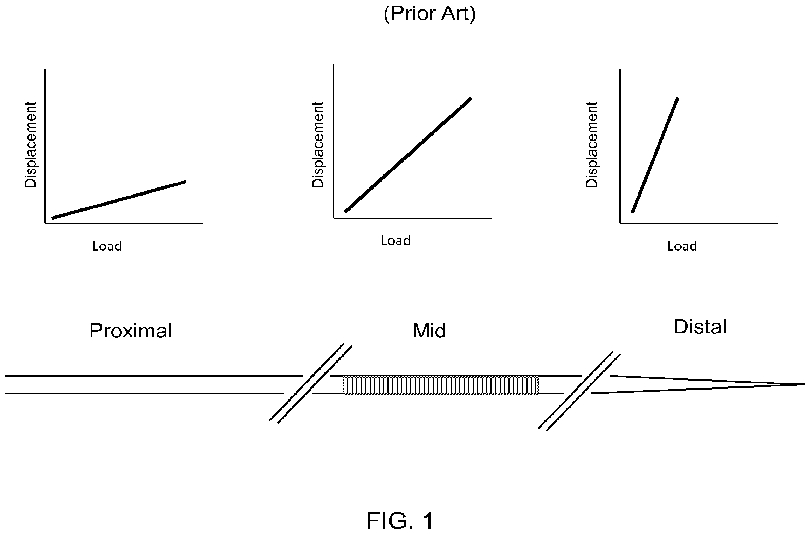

Contemporary intraluminal devices (e.g., catheters) have attempted to achieve a balance between stiffness and flexibility by adding a material of intermediate stiffness between the proximal and distal ends of the device, as shown in FIG. 1. FIG. 1 depicts the general properties of the various sections of an intraluminal device, e.g., a catheter, using this concept. The proximal end is relatively stiff, shown graphically as a small correlation between load and lateral displacement. In other words, when transverse force is exerted on the proximal end, the proximal end only flexes a small amount. In contrast, the distal end is quite flexible and experiences a large amount of lateral displacement with a relatively small amount of applied load.

In order to reduce kinking in the device, most intraluminal devices add a material of intermediate stiffness between the proximal and distal ends, shown in FIG. 1 as an intermediate graphical relationship between load and lateral displacement. A variety of different materials are used to achieve the desired mechanical properties of the proximal, mid, and distal portions. For example, the proximal end of many intraluminal devices includes a stiff hollow tube (hypotube) that is only slightly flexible and has excellent compressional strength, allowing a physician to deliver force laterally along the catheter. Hypotubes may be constructed from standard metals, such as stainless steel, or from memory metals, such as nitinol, an alloy of nickel and titanium. Hypotubes may also be constructed from polymers such as PEBAX®, nylon, HDPE, and PEEK. The distal end of the intraluminal device is typically constructed from a flexible polymer with good kink resistance, such as a fluoropolymer. Midsections are typically constructed from polymers with better stiffness, such as polyamides, to provide transitional flexibility between the proximal and distal ends.

Contemporary designs with intermediate stiffness midsections perform better than earlier intraluminal devices, which essentially comprised a metal tube (proximal end) connected to a plastic tube (distal end). Designs with intermediate midsections of a single material still have shortcomings, however. The transition between the midsection and the distal end is prone to kinking because of the abrupt transition in materials properties. Kinking in a balloon angioplasty catheter can harm a patient because the kink reduces the rate at which the inflation fluid can be removed from the balloon. If the balloon stays inflated for more than two minutes, downstream tissues will be damaged due to hypoxia. Kinking can also damage imaging catheters, especially rotary pullback catheters, if the signal or drive cables are compromised. Furthermore, even slight amounts of kinking can cause image distortions in rotational imaging systems because the imaging element does not track the rotational force exerted at the proximal end, leading to nonuniform rotational distortion (NURD) in the images.

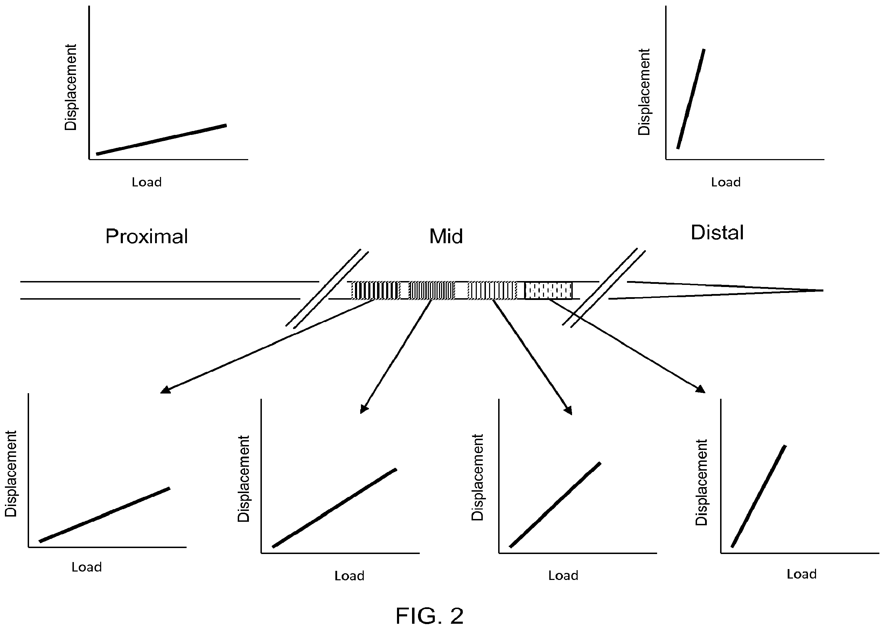

Intraluminal devices of the invention overcome the shortcomings of the prior art by providing a variable stiffness midsection that more effectively transitions the intraluminal device from a stiff proximal end to a flexible distal end. As depicted graphically in FIG. 2, the variable stiffness midsection is actually a series of variable mechanical properties, giving rise to a more gradual transition in the relationship between load and lateral displacement, as shown in the series of graphs at the bottom of FIG. 2. The desired transition in stiffness through the midsection can be achieved by coupling various materials with different mechanical properties, by using tapered materials that allow overlap between elements with different properties, or by using specialty materials that are designed to have the desired transitional properties.

The concepts of the invention may be applied to any intraluminal device. In some embodiments, the intraluminal device is a catheter. A variety of intravascular catheters are known. In practice, intravascular catheters are delivered to a tissue of interest via an introducer sheath placed in the radial, brachial or femoral artery. The introducer is inserted into the artery with a large needle, and after the needle is removed, the introducer provides access for guide wires, catheters, and other endovascular tools. An experienced cardiologist can perform a variety of procedures through the introducer by inserting tools such as balloon catheters, stents, or cauterization instruments. When the procedure is complete the introducer is removed, and the wound can be secured with suture tape. Catheter lengths vary up to 400 cm, depending on the anatomy and work flow.

In some embodiments, the catheter will be an imaging or sensing catheter. Imaging catheters allow a physician to acquire images of tissues from within a lumen, e.g., a blood vessel. Often it is instructive to image a tissue prior to treatment, e.g., with angioplasty or drugs. The image may be obtained with acoustic waves, i.e., ultrasound, or the image may be obtained with light. Imaging catheters may include any of a number of known imaging modalities, such as intravascular ultrasound (IVUS) imaging or optical coherence tomography (OCT). The IVUS imaging assembly may be phased array IVUS imaging assembly, a pull-back type IVUS imaging assembly, or an IVUS imaging assembly that uses photoacoustic materials to produce diagnostic ultrasound and/or receive reflected ultrasound for diagnostics. IVUS imaging assemblies and processing of IVUS data are described for example in Yock, U.S. Pat. Nos. 4,794,931, 5,000,185, and 5,313,949; Sieben et al., U.S. Pat. Nos. 5,243,988, and 5,353,798; Crowley et al., U.S. Pat. No. 4,951,677; Pomeranz, U.S. Pat. No. 5,095,911, Griffith et al., U.S. Pat. No. 4,841,977, Maroney et al., U.S. Pat. No. 5,373,849, Born et al., U.S. Pat. No. 5,176,141, Lancee et al., U.S. Pat. No. 5,240,003, Lancee et al., U.S. Pat. No. 5,375,602, Gardineer et at., U.S. Pat. No. 5,373,845, Seward et al., Mayo Clinic Proceedings 71(7):629-635 (1996), Packer et al., Cardiostim Conference 833 (1994), "Ultrasound Cardioscopy," Eur. J.C.P.E. 4(2):193 (June 1994), Eberle et al., U.S. Pat. No. 5,453,575, Eberle et al., U.S. Pat. No. 5,368,037, Eberle et at., U.S. Pat. No. 5,183,048, Eberle et al., U.S. Pat. No. 5,167,233, Eberle et at., U.S. Pat. No. 4,917,097, Eberle et at., U.S. Pat. No. 5,135,486, and other references well known in the art relating to intraluminal ultrasound devices and modalities.

In other embodiments, the imaging may use optical coherence tomography (OCT). OCT is a medical imaging methodology using a miniaturized near infrared light-emitting probe, and is capable of acquiring micrometer-resolution, three-dimensional images from within optical scattering media (e.g., biological tissue). OCT systems and methods are generally described in Castella et al., U.S. Patent No. 8,108,030, Milner et al., U.S. Patent Application Publication No. 2011/0152771, Condit et al., U.S. Patent Application Publication No. 2010/0220334, Castella et al., U.S. Patent Application Publication No. 2009/0043191, Milner et al., U.S. Patent Application Publication No. 2008/0291463, and Kemp, N., U.S. Patent Application Publication No. 2008/0180683.

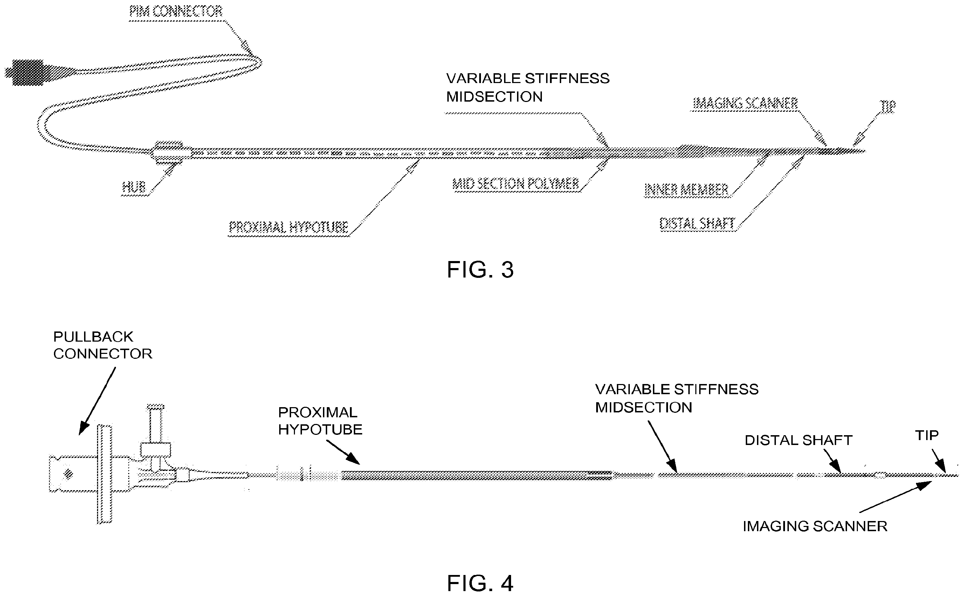

An exemplary phased-array IVUS catheter having a variable stiffness midsection is shown in FIG. 3. An exemplary rotational pullback IVUS catheter having a variable stiffness midsection is shown in FIG. 4. The improved midsection is not immediately evident in the exterior view of either catheter, because the variable stiffness midsection is designed to integrate into the catheter without producing seams or other topography that could interact with material (e.g., plaque) in the vasculature. Additionally, while not shown, the imaging catheters of FIGS. 3 and 4 comprise lubricous coatings on the exterior to facilitate movement through the vasculature.

Catheters of the invention may be configured to allow the passage of rotary drive shafts, as needed for pullback IVUS and OCT. The rotary drive shaft permits transmission of torque from the rotary motor via the signal train along the entire length of the catheter. Because the drive shaft is coupled to the imaging element, translation of the drive shaft will result in translation of the imaging element. Typically, the drive shafts are microcables, i.e., smaller than 5 mm in diameter, e.g., smaller than 4 mm in diameter, e.g., smaller than 3 mm in diameter, e.g., smaller than 2 mm in diameter. Such a rotary drive shaft is typically concentrically- or coaxially-positioned within the central lumen of the catheter, i.e., a catheter having a variable stiffness mid-section, as described below. In some embodiment, the signal train (electronic signal or optical signal) is carried by the rotary drive shaft with a signal wire or an optical fiber running the length of the rotary drive shaft. The rotary drive shaft may include one or more signal coupling elements to assure uninterrupted communication between the imaging/detection elements and the proximal end of the catheter. Such coupling elements are configured to allow free rotation and continuous passage of light or electrical signal.

Alternatively, or in addition to imaging, catheters of the invention can also be used to evaluate and provide therapy to vascular tissues (i.e., blood vessels) as well as other body lumens. Thus, catheters of the invention allow a variety of treatments to be administered, including, but not limited to drug delivery, energy therapy (e.g., light or acoustic), aspiration, ablation, angioplasty, debulking, or implant delivery (stent, filter, valve). For example, the invention includes drug delivery catheters that are capable of IVUS imaging and Doppler flow monitoring. Accordingly, catheters of the invention may include one or more tool lumens, formed from an inner catheter sheath or member that is disposed within the inner body of the catheter. Through the tool lumen, a catheter tool or device can be introduced into a body lumen, such as blood vessel, for treatment. In addition, the catheter may optionally include a removal lumen that extends from the distal end of the imaging catheter to an opening operably associated with a vacuum source. During intraluminal procedures, for example, a tool element may shave off plaque or other substances from the vessel wall that needs to be removed from the lumen. The shaved-off plaque can be removed from the removal lumen.

In other embodiments, a catheter of the invention may include ablation capabilities. In some instances, the ablation tool can be extended from the catheter lumen and into a vessel, such as a blood vessel, to perform ablation therapy. Additionally, the catheter may provide imaging to allow the field of therapy to be observed before, during, and after the ablation therapy. For example, a therapy catheter can be configured to image the ablation procedure performed along the side of the catheter while imaging the treatment tissue with a proximal (or distal) imaging element. There are several different types of ablation therapies. In one aspect, an ablation tool is used to remove an unwanted or damaged vein by delivering energy (RF energy, laser energy, etc.) within a vein to shrink and ultimately close the vein. In another aspect, an ablation tool is used to treat heart arrhythmia disorders by ablating abnormal heart tissue to create scar tissue and disrupt the conduction pathway that lead to the disruption. In another example, the ablation tool is used to perform an atherectomy procedure to ablate arethoma or plaque within a vessel. Arethoma is an accumulation and swelling in artery walls made up of (mostly) macrophage cells, or debris, and containing lipids (cholesterol and fatty acids), calcium and a variable amount of fibrous connective tissue. In some instances, the proximal end of the ablation tool is connected to an energy source that provides energy to the electrodes for ablation. The energy necessary to ablate cardiac tissue and create a permanent lesion can be provided from a number of different sources including radiofrequency, laser, microwave, ultrasound and forms of direct current (high energy, low energy and fulgutronization procedures). Any source of energy is suitable for use in the ablation tool of the invention. In some embodiments, the ablation tool includes at least one electrode. The electrodes can be arranged in many different patterns along the ablation tool. For example, the electrode may be located on a distal end of the ablation tool. In addition, the electrodes may have a variety of different shape and sizes. For example, the electrode can be a conductive plate, a conductive ring, conductive loop, or a conductive coil. In one embodiment, the at least one electrode includes a plurality of wire electrodes configured to extend out of the distal end of the imaging electrode.

In other embodiments, the catheter includes implant delivery mechanism(s). The implant delivery mechanism may be configured to deploy an implant into the lumen of a body vessel, such as a blood vessel. The implant or device may be placed in the vessel permanently/long term or temporarily/short term purposes. Implants can be placed at the treatment site (such as stents) or implants can be placed near the treatment site to occlude or filter the vessel (such as plugs or filters). Additionally, delivery catheters of the invention can be configured with imaging, to allow the treatment field to be imaged prior to, during, and after implantation. For example, an imaging element of the catheter may be used to locate the implant placement site and to position the catheter for implant delivery. During implant delivery, the imaging catheter can, for example, image a stent being deployed distally from the guide wire. In addition, a combined imaging and delivery catheter prevents the need to exchange a delivery catheter for an imaging catheter, thus decreasing operation time.

In other embodiments the intraluminal device will be a guide wire. Guide wires are known medical devices used in the vasculature or other anatomical passageway and act as a guide for other devices, e.g., a catheter. Typically, the guide wire is inserted into an artery or vein and guided through the vasculature under fluoroscopy (real time x-ray imaging) to the location of interest. Guide wires typically have diameters of 0.010" (0.0254 cm) to 0.035" (0.0889 cm), with 0.014" (0.03556 cm) being the most common. Guide wires (and other intravascular objects) are also sized in units of French, each French being 1/3 of a mm or 0.013". Guide wire lengths vary up to 400 cm, depending on the anatomy and work flow. Often a guide wire has a flexible distal tip portion about 3 cm long and a slightly less flexible portion about 30 to 50 cm long leading up to the tip with the remainder of the guide wire being stiffer to assist in maneuvering the guide wire through tortuous vasculature, etc. The tip of a guide wire typically has a stop or a hook to prevent a guided device, e.g., a catheter from passing beyond the distal tip. In some embodiments, the tip can be deformed by a user to produce a desired shape. The invention can be used with advanced guide wire designs, include guide wires that use sensors that measure flow and pressure, among other things. For example, the FLOWIRE® Doppler Guide Wire, available from Volcano Corp. (San Diego, CA), has a tip-mounted ultrasound transducer and can be used in all blood vessels, including both coronary and peripheral vessels, to measure blood flow velocities during diagnostic angiography and/or interventional procedures.

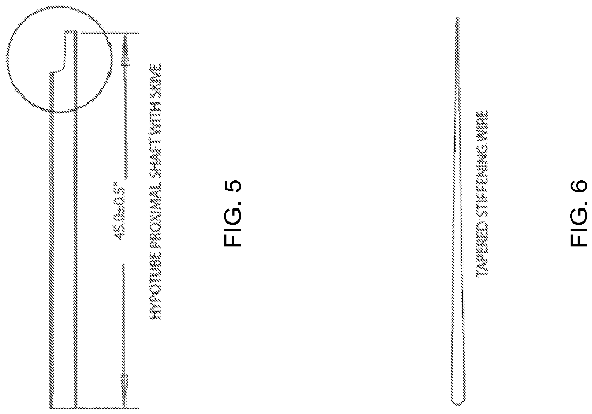

A first embodiment of a structure for providing a variable stiffness element is shown in FIGS. 5-8B. The embodiment may be loosely summarized as a tapered wire bonded to a proximal hypotube. FIG. 5 shows a metal hypotube 500 with a skived distal end 510, used to form the proximal portion of a catheter. The hypotube 500 may be fabricated from metal or plastic, as discussed previously. Hypotubes having the design shown in FIG. 5 are available from custom hypotube manufacturers such as Johnson Matthey Medical Components (West Chester, PA).

The embodiment additionally comprises a tapered wire 600, shown in FIG. 6. The tapered wire 600 may be constructed from a number of different metals to achieve the desired strength and stiffness. The tapered wire 600 may be formed of surgical stainless steel (316L, 316LVM), nickel, tungsten, zinc, copper, or alloys thereof. The tapered wire 600 may be formed of a shape alloy, such as nitinol.

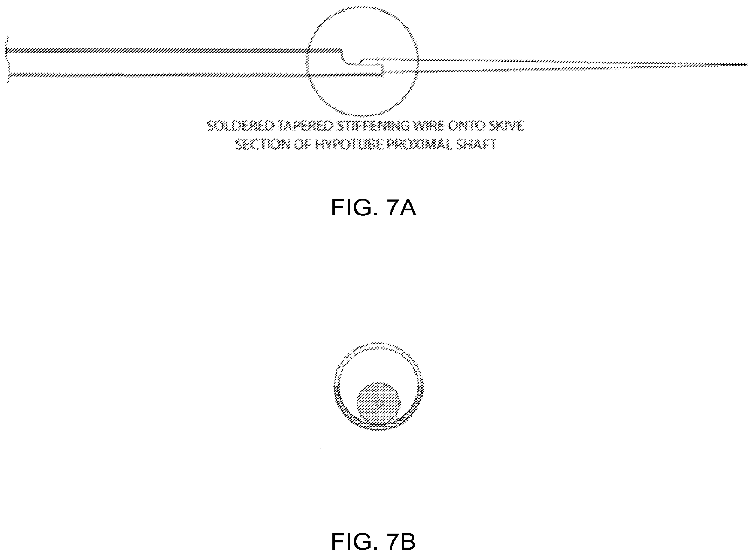

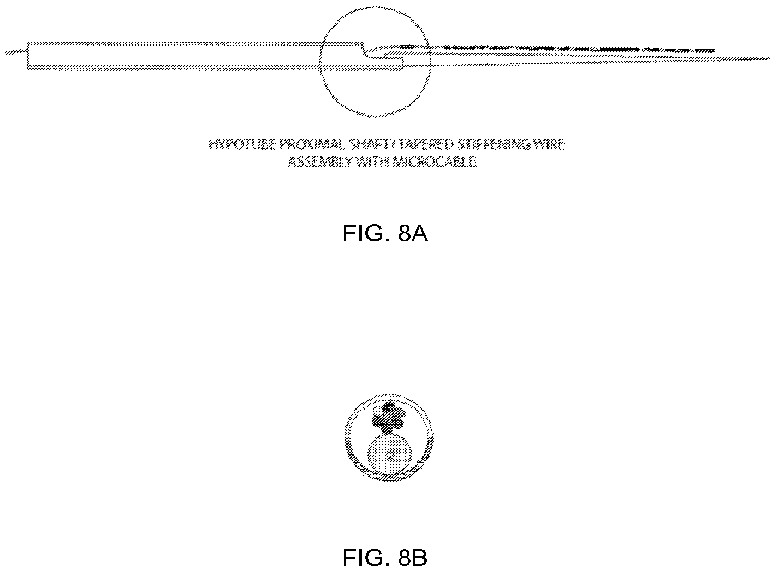

To produce the variable stiffness element, the tapered stiffening wire 600 is joined with the hypotube 500. The tapered stiffening wire 600 and hypotube 500 may be joined with microwelding, solder, or epoxy, as shown in FIGS. 7A and 7B. Once completed, a microcable 800 can be passed through the hypotube 500 to connect with an interface at the proximal end of the catheter, as shown in FIG. 8A. As shown in FIG. 8B, the microcable 800 passes comfortably over the tapered stiffening wire 600 and through the hypotube 500. The microcable 800 can be used to deliver power to the distal end of the device, return signals from the distal end of the device, provide translational and rotational motion to an imaging device, or a combination thereof.

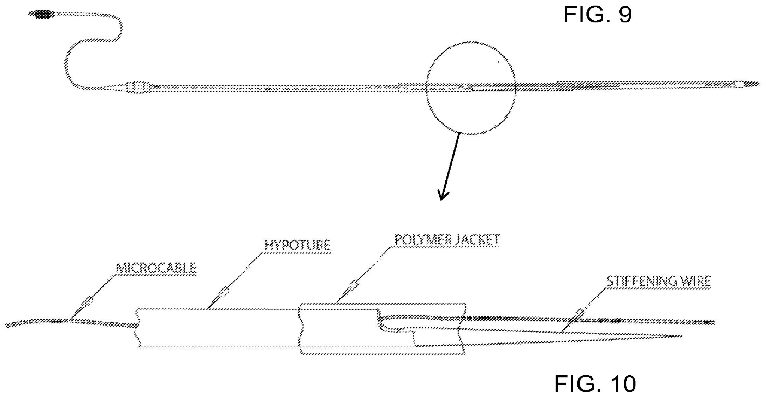

An imaging catheter 900, incorporating a variable stiffness element of FIGS. 7 and 8, is shown in FIG. 9. The circled segment of catheter 900, corresponding to a variable stiffness midsection assembly 1000, is shown in greater detail in FIG. 10. The variable stiffness midsection assembly 1000 includes the hypotube 500 soldered to stiffening wire 600 and microcable 800. The entire assembly 1000 is covered in a polymer jacket 700, which provides a smooth transition along the exterior surface of catheter 900.

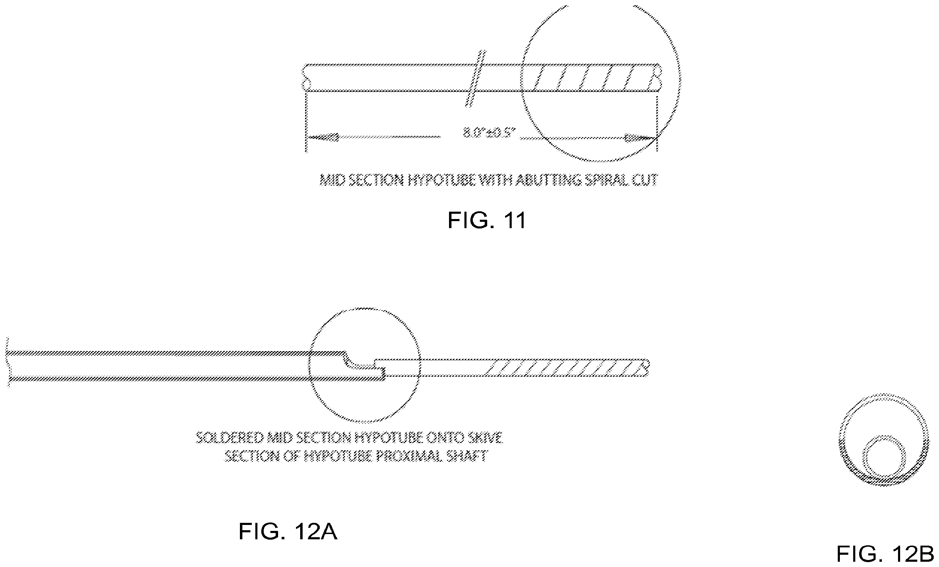

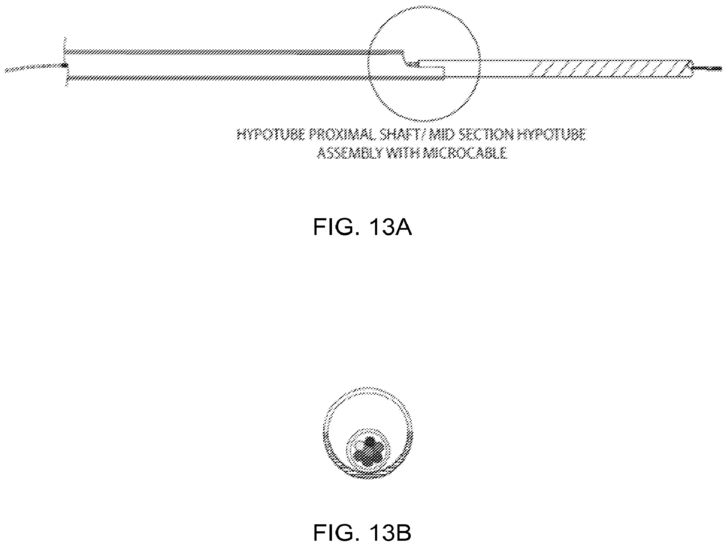

A second embodiment of a structure for providing a variable stiffness element is shown in FIGS. 11-13B. The embodiment may be loosely summarized as a spiral-cut hypotube bonded to a proximal hypotube. FIG. 11 shows a spiral-cut hypotube 1100 which will provide flexibility to the midsection of a catheter. The spiral-cut hypotube 1100 may be fabricated from metal or plastic, as discussed previously. To produce the variable stiffness element, the spiral-cut hypotube 1100 is joined with the hypotube 500. The spiral-cut hypotube 1100 and hypotube 500 may be joined with microwelding, solder, or epoxy, as shown in FIGS. 12A and 12B. Once completed, a microcable 800 can be passed through the spiral-cut hypotube 1100 and the hypotube 500 to connect with an interface at the proximal end of the catheter, as shown in FIG. 13A. As shown in FIG. 13B, the microcable 800 passes comfortably through the spiral-cut hypotube 1100 and through the hypotube 500. The microcable 800 can be used to deliver power to the distal end of the device, return signals from the distal end of the device, provide translational and rotational motion to an imaging device, or a combination thereof.

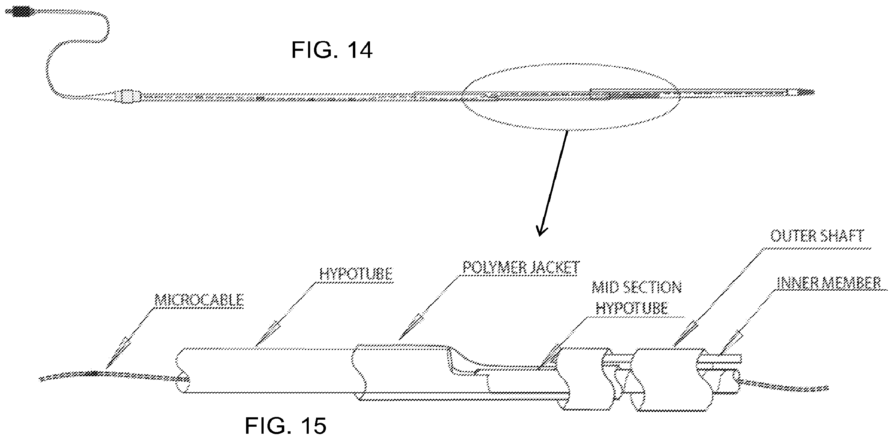

An imaging catheter 1400, incorporating a variable stiffness element of FIGS. 12 and 13, is shown in FIG. 14. The circled segment of catheter 1400, corresponding to a variable stiffness midsection assembly 1500, is shown in greater detail in FIG. 15. The variable stiffness midsection assembly 1500 includes the hypotube 500 soldered to the spiral-cut hypotube 1100 and microcable 800. The entire assembly 1500 is covered in a polymer jacket 700, which provides a smooth transition along the exterior surface of catheter 1400. In the embodiment shown in FIG. 15, the catheter 1400 additionally includes an outer shaft 1510 and an inner member 1520 which provide a housing for the imaging assembly (not shown).

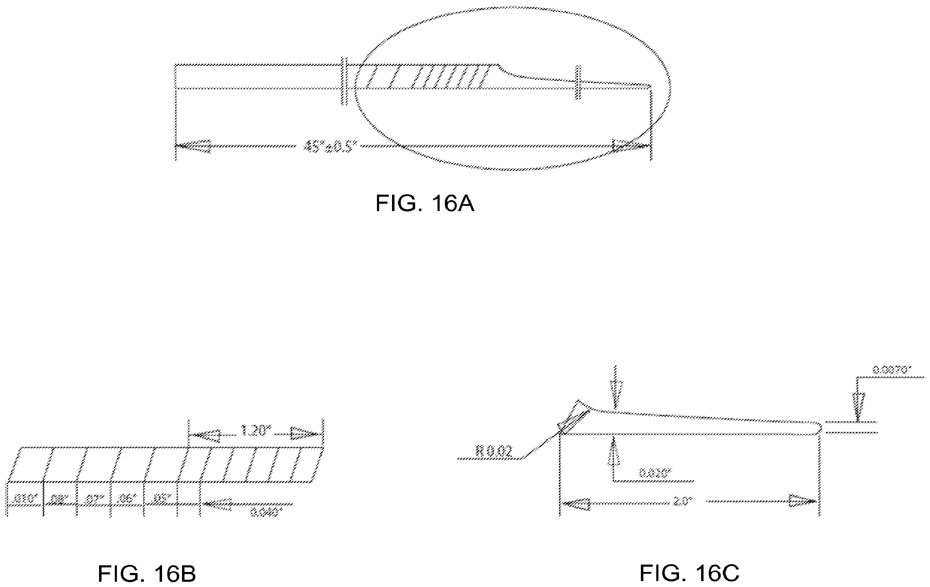

A third embodiment of a structure for providing a variable stiffness element is shown in FIGS. 16-17B. The embodiment may be loosely summarized as an extended hypotube having a spiral-cut and an extended skive. FIG. 16A shows a side-view of an extended hypotube with a spiral cut 1600 which will provide stiffness to the proximal end of a catheter in addition to flexibility to the midsection of a catheter. The extended hypotube with a spiral cut 1600 comprises a tubular section 1610, a spiral cut section 1620, and an elongated skive section 1630. The spiral-cut section 1620 is shown in greater detail in FIG. 16B. The spacing of the spiral in the spiral-cut section 1620 may be constant, or it may vary, as shown in FIG. 16B. The elongated skive section 1630 is shown in greater detail in FIG. 16C. The extended hypotube with a spiral cut 1600 may be fabricated from metal or plastic, as discussed previously. In one embodiment, the extended hypotube with a spiral cut 1600 has an outer diameter of less than 0.5 cm, the tubular section 1610 has a length of at least 10 cm, the elongated skive section 1630 has a length of at least 2 cm, and the spiral-cut section 1620 has a length of at least 1 cm.

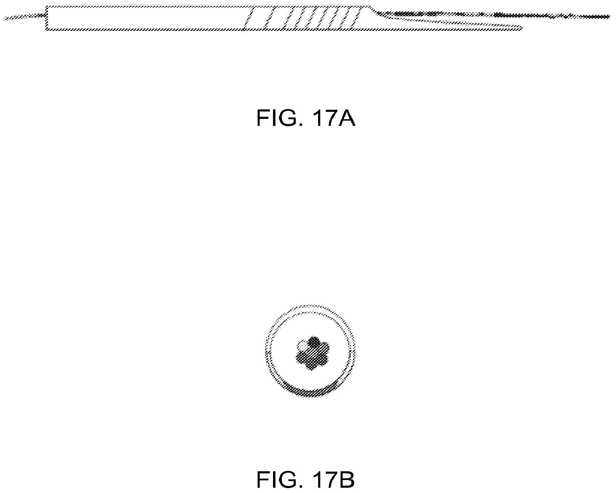

A microcable 800 can be passed through the extended hypotube with a spiral cut 1600 to connect with an interface at the proximal end of the catheter, as shown in FIG. 17A. As shown in FIG. 17B, the microcable 800 passes comfortably through the extended hypotube with a spiral cut 1600. The microcable 800 can be used to deliver power to the distal end of the device, return signals from the distal end of the device, provide translational and rotational motion to an imaging device, or a combination thereof.

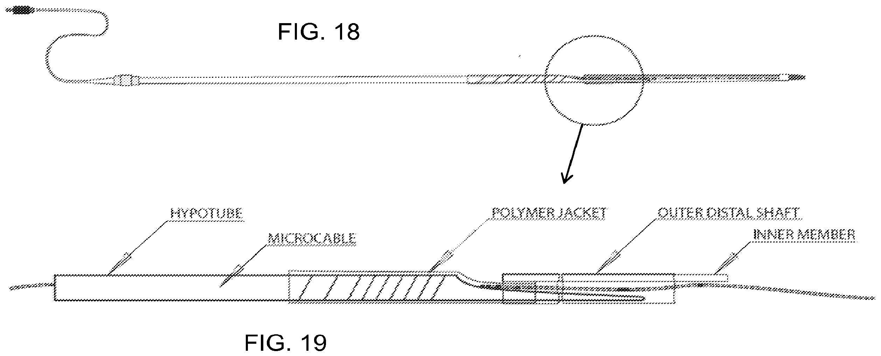

An imaging catheter 1800, incorporating a variable stiffness element of FIGS. 16 and 17, is shown in FIG. 18. The circled segment of catheter 1800, corresponding to a variable stiffness midsection assembly 1900, is shown in greater detail in FIG. 19. The variable stiffness midsection assembly 1900 includes the extended hypotube with a spiral cut 1600 and microcable 800. The entire assembly 1900 is covered in a polymer jacket 700, which provides a smooth transition along the exterior surface of catheter 1800. In the embodiment shown in FIG. 19, the catheter 1900 additionally includes an outer shaft 1510 and an inner member 1520 which provide a housing for the imaging assembly (not shown).

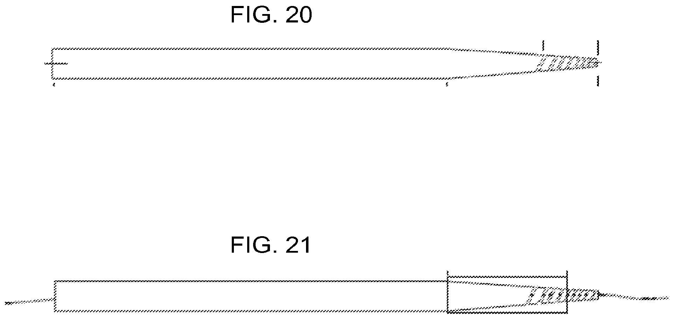

A fourth embodiment of a structure for providing a variable stiffness element is shown in FIGS. 20 and 21. The embodiment may be loosely summarized as an extended hypotube having tapered and spiral-cut segments. FIG. 20 shows a side-view of an extended hypotube having tapered and spiral-cut segments 2000 which will provide stiffness to the proximal end of a catheter in addition to flexibility to the midsection of a catheter. The extended hypotube having tapered and spiral-cut segments 2000 comprises a tubular section 2010, a tapered section 2020, and a spiral cut section 2030. The spacing of the spiral in the spiral-cut section 2030 may be constant, or it may vary. The extended hypotube having tapered and spiral-cut segments 2000 may be fabricated from metal or plastic, as discussed previously. In one embodiment, the extended hypotube having tapered and spiral-cut segments 2000 has an outer diameter of less than 0.5 cm, the tubular section 2010 has a length of at least 10 cm, the spiral-cut section 2030 has a length of at least 1 cm, and the tapered section 2020 has length of at least 1 cm.

A microcable 800 can be passed through the extended hypotube having tapered and spiral-cut segments 2000 to connect with an interface at the proximal end of the catheter, as shown in FIG. 21. The microcable 800 passes comfortably through the extended hypotube having tapered and spiral-cut segments 2000. The microcable 800 can be used to deliver power to the distal end of the device, return signals from the distal end of the device, provide translational and rotational motion to an imaging device, or a combination thereof.

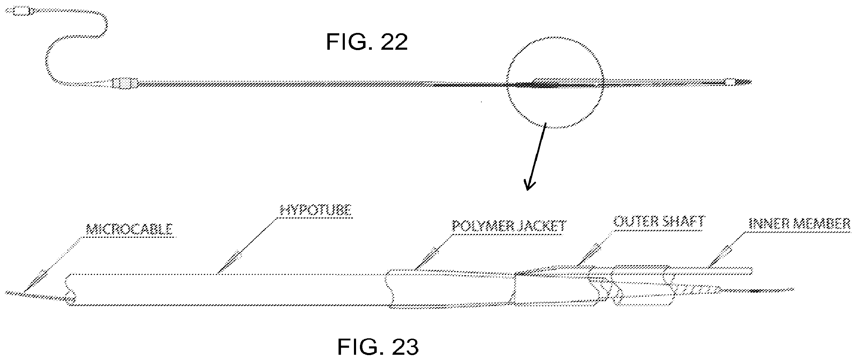

An imaging catheter 2200, incorporating a variable stiffness element of FIG. 20, is shown in FIG. 23. The circled segment of catheter 2200, corresponding to a variable stiffness midsection assembly 2300, is shown in greater detail in FIG. 23. The variable stiffness midsection assembly 2300 includes the extended hypotube having tapered and spiral-cut segments 2000 and microcable 800. The entire assembly 2300 is covered in a polymer jacket 700, which provides a smooth transition along the exterior surface of catheter 2200. In the embodiment shown in FIG. 23, the catheter 2200 additionally includes an outer shaft 1510 and an inner member 1520 which provide a housing for the imaging assembly (not shown).

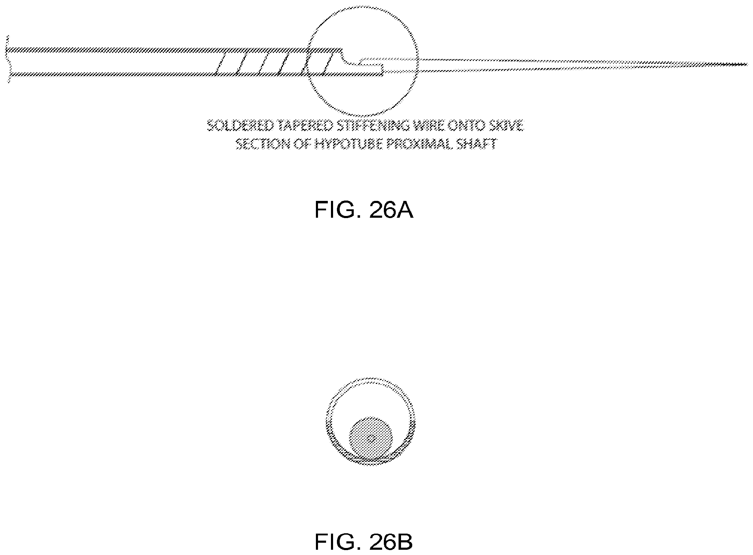

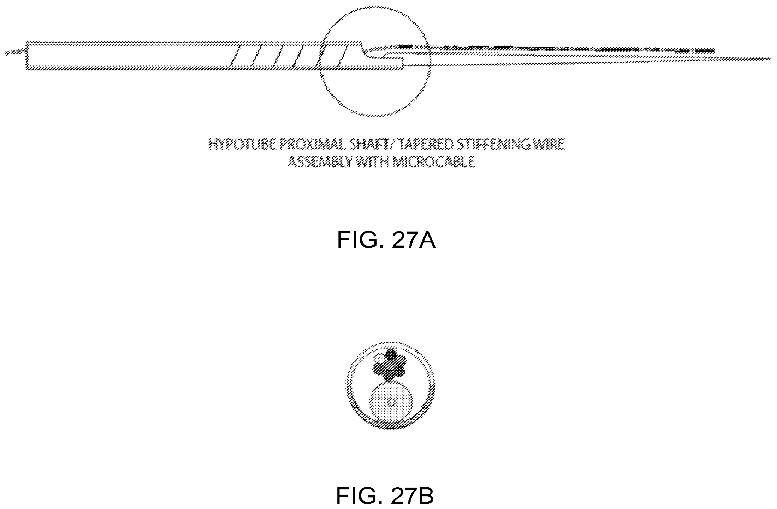

A fifth embodiment of a structure for providing a variable stiffness element is shown in FIGS. 23-26B. The embodiment may be loosely summarized as a spiral-cut hypotube bonded to a tapered stiffening wire. FIG. 23 shows a side-view of a proximal hypotube with a spiral cut 2400 which will provide stiffness to the proximal end of a catheter in addition to flexibility to the midsection of a catheter. The proximal hypotube with a spiral cut 2400 may be fabricated from metal or plastic, as discussed previously. To produce the variable stiffness element, proximal hypotube with a spiral cut 2400 is joined with the tapered stiffening wire, discussed previously. The proximal hypotube with a spiral cut 2400 and the tapered stiffening wire 600 may be joined with microwelding, solder, or epoxy, as shown in FIGS. 26A and 26B. Once completed, a microcable 800 can be passed through the proximal hypotube with a spiral cut 2400 and over the tapered stiffening wire 600 to connect with an interface at the proximal end of the catheter, as shown in FIG. 27A. As shown in FIG. 27B, the microcable 800 passes comfortably through the proximal hypotube with a spiral cut 2400 and over the tapered stiffening wire 600. The microcable 800 can be used to deliver power to the distal end of the device, return signals from the distal end of the device, provide translational and rotational motion to an imaging device, or a combination thereof.

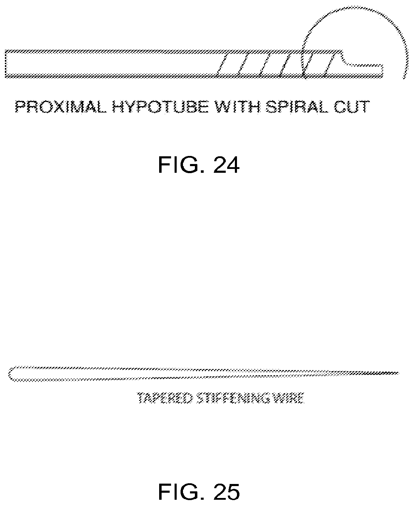

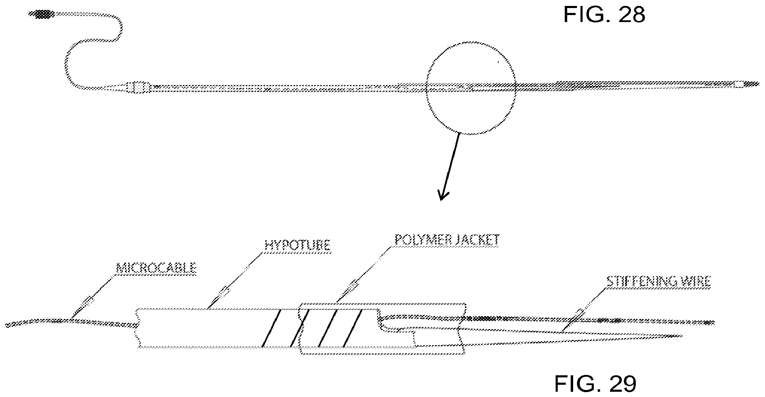

An imaging catheter 2800, incorporating a variable stiffness element of FIGS. 24-27B, is shown in FIG. 28. The circled segment of catheter 2800, corresponding to a variable stiffness midsection assembly 2900, is shown in greater detail in FIG. 29. The variable stiffness midsection assembly 2900 includes the proximal hypotube with a spiral cut 2400 soldered to the tapered stiffening wire 600 and microcable 800. The entire assembly 1500 is covered in a polymer jacket 700, which provides a smooth transition along the exterior surface of catheter 1400.

Using the devices of the invention, a variety of target tissues can be imaged, diagnosed, treated, and evaluated with the devices of the invention. In particular the invention is useful for treating tissues that are accessible via the various lumina of the body, including, but not limited to, blood vessels, vasculature of the lymphatic and nervous systems, structures of the gastrointestinal tract (lumina of the small intestine, large intestine, stomach, esophagus, colon, pancreatic duct, bile duct, hepatic duct), lumina of the reproductive tract (vas deferens, uterus and fallopian tubes), structures of the urinary tract (urinary collecting ducts, renal tubules, ureter, and bladder), and structures of the head and neck and pulmonary system (sinuses, parotid, trachea, bronchi, and lungs). Accordingly, the devices of the invention may be beneficial in the treatment of a number of disorders, including, but not limited to, atherosclerosis, ischemia, coronary blockages, thrombi, occlusions, stenosis, and aneurysms. The devices can also be used to treat cancer, inflammatory disease (e.g., autoimmune disease, arthritis), pain, and genetic disorders.

## Claims

1. An imaging catheter (900, 1400) comprising a proximal segment (500), a distal segment and a midsection (1000) there between, the proximal segment comprising a hypotube comprising a skived terminal portion, the distal segment comprising an imaging element, and the midsection comprising a variable stiffness element, wherein:
the variable stiffness element comprises a tapered wire (600) and the tapered wire is bonded to the skived terminal portion (510) of the hypotube, or
the variable stiffness element comprises a spiral-cut tube (1100) and the spiral cut tube is bonded to the skived terminal portion of the hypotube.

2. The imaging catheter of claim 1, wherein the hypotube comprises a metal.

3. The imaging catheter of claim 2, wherein the metal comprises stainless steel or nitinol.

4. The imaging catheter of claim 1, wherein the hypotube comprises a polymer.

5. The imaging catheter of claim 1, wherein the hypotube comprises a spiral cut (2400).

6. The imaging catheter of claim 1, wherein the tapered wire comprises stainless steel or nitinol.

7. The imaging catheter of claim 1, wherein the tapered wire is soldered to the hypotube.

8. The imaging catheter of claim 1, wherein the spiral-cut tube comprises stainless steel or nitinol.

9. The imaging catheter of claim 1, additionally comprising a microcable (800) spanning a portion of the catheter and transitioning through an interior of the hypotube.

10. The imaging catheter of claim 9, wherein the microcable provides torque and translation to the imaging element.

11. The imaging catheter of claim 1, additionally comprising an optical fiber spanning a portion of the catheter and transitioning through an interior of the hypotube.

12. The imaging catheter of claim 1, further comprising an ultrasound transducer.

13. The imaging catheter of claim 12, wherein the ultrasound transducer can be translated within a distal segment of the catheter.

14. The imaging catheter of claim 1, wherein the proximal segment is stiffer than the distal segment and the midsection provides gradated intermediate stiffness from the relatively stiff proximal section to the relatively flexible distal segment.

## Patentansprüche

1. Bildgebungskatheter (900, 1400), umfassend ein proximales Segment (500), ein distales Segment und einen Mittelabschnitt (1000) dazwischen, wobei das proximale Segment ein Hypotube umfasst, das einen ausgeschärften Endabschnitt umfasst, das distale Segment ein Bildgebungselement umfasst und der Mittelabschnitt ein Element variabler Steifigkeit umfasst, wobei:
das Element variabler Steifigkeit einen verjüngten Draht (600) umfasst und der verjüngte Draht an den ausgeschärften Endabschnitt (510) des Hypotubes gebunden ist oder
das Element variabler Steifigkeit ein spiralförmig geschnittenes Rohr (1100) umfasst und das spiralförmig geschnittene Rohr an den ausgeschärften Endabschnitt des Hypotubes gebunden ist.

2. Bildgebungskatheter nach Anspruch 1, wobei das Hypotube ein Metall umfasst.

3. Bildgebungskatheter nach Anspruch 2, wobei das Metall Edelstahl oder Nitinol umfasst.

4. Bildgebungskatheter nach Anspruch 1, wobei das Hypotube ein Polymer umfasst.

5. Bildgebungskatheter nach Anspruch 1, wobei das Hypotube einen Spiralschnitt (2400) umfasst.

6. Bildgebungskatheter nach Anspruch 1, wobei der verjüngte Draht Edelstahl oder Nitinol umfasst.

7. Bildgebungskatheter nach Anspruch 1, wobei der verjüngte Draht an das Hypotube gelötet ist.

8. Bildgebungskatheter nach Anspruch 1, wobei das spiralförmig geschnittene Rohr Edelstahl oder Nitinol umfasst.

9. Bildgebungskatheter nach Anspruch 1, zusätzlich umfassend ein Mikrokabel (800), das einen Teil des Katheters überspannt und durch ein Inneres des Hypotubes verläuft.

10. Bildgebungskatheter nach Anspruch 9, wobei das Mikrokabel dem Bildgebungselement ein Drehmoment und Verschiebung verleiht.

11. Bildgebungskatheter nach Anspruch 1, zusätzlich umfassend eine optische Faser, die einen Teil des Katheters überspannt und durch ein Inneres des Hypotubes verläuft.

12. Bildgebungskatheter nach Anspruch 1, weiter umfassend einen Ultraschallwandler.

13. Bildgebungskatheter nach Anspruch 12, wobei der Ultraschallwandler innerhalb eines distalen Segments des Katheters verschoben werden kann.

14. Bildgebungskatheter nach Anspruch 1, wobei das proximale Segment steifer ist als das distale Segment und der Mittelabschnitt eine abgestufte Zwischensteifigkeit von dem relativ steifen proximalen Abschnitt zu dem relativ flexiblen distalen Segment bereitstellt.

## Revendications

1. Cathéter d'imagerie (900, 1400) comprenant un segment proximal (500), un segment distal et une section médiane (1000) entre ceux-ci, le segment proximal comprenant un hypotube comprenant une partie terminale refendue, le segment distal comprenant un élément d'imagerie, et la section médiane comprenant un élément à rigidité variable, dans lequel :
l'élément à rigidité variable comprend un fil effilé (600) et le fil tronconique est collé à la partie terminale refendue (510) de l'hypotube, ou
l'élément à rigidité variable comprend un tube coupé en spirale (1100) et le tube coupé en spirale est collé à la partie terminale refendue de l'hypotube.

2. Cathéter d'imagerie selon la revendication 1, dans lequel l'hypotube comprend un métal.

3. Cathéter d'imagerie selon la revendication 2, dans lequel le métal comprend de l'acier inoxydable ou du nitinol.

4. Cathéter d'imagerie selon la revendication 1, dans lequel l'hypotube comprend un polymère.

5. Cathéter d'imagerie selon la revendication 1, dans lequel l'hypotube comprend une coupe en spirale (2400).

6. Cathéter d'imagerie selon la revendication 1, dans lequel le fil conique comprend de l'acier inoxydable ou du nitinol.

7. Cathéter d'imagerie selon la revendication 1, dans lequel le fil effilé est soudé à l'hypotube.

8. Cathéter d'imagerie selon la revendication 1, dans lequel le tube coupé en spirale comprend de l'acier inoxydable ou du nitinol.

9. Cathéter d'imagerie selon la revendication 1, comprenant en outre un micro-câble (800) s'étendant sur une partie du cathéter et effectuant une transition à travers un intérieur de l'hypotube.

10. Cathéter d'imagerie selon la revendication 9, dans lequel le micro-câble fournit un couple et une translation à l'élément d'imagerie.

11. Cathéter d'imagerie selon la revendication 1, comprenant en outre une fibre optique s'étendant sur une partie du cathéter et effectuant une transition à travers un intérieur de l'hypotube.

12. Cathéter d'imagerie selon la revendication 1, comprenant en outre un transducteur à ultrasons.

13. Cathéter d'imagerie selon la revendication 12, dans lequel le transducteur à ultrasons peut être déplacé en translation dans un segment distal du cathéter.

14. Cathéter d'imagerie selon la revendication 1, dans lequel le segment proximal est plus rigide que le segment distal et la section médiane fournit une rigidité intermédiaire graduée de la section proximale relativement rigide au segment distal relativement flexible.
